# EUROPEAN PATENT APPLICATION

(11) **EP 2 145 581 A1**
(43) Date of publication of application: **20.01.2010**
(21) Application number: 09164906.1
(22) Date of filing: 08.07.2009
(51) Int. Cl.: A61B 5/00

(54) **System for providing remote signals from a patient monitor**

(30) Priority: 17.07.2008 US 218748
(71) Applicant: General Electric Company, Schenectady, NY 12345 (US)
(72) Inventor: Reed, Chad M., Pendleton, IN 46064-8698 (US)
(74) Representative: Illingworth-Law, William Illingworth

(57) **Abstract**

A system for providing remote audible and /or visible signals corresponding to those of a patient monitor (10). The patient monitor (10) is locate proximate to the patient and senses certain conditions of the patient and provides visible and/or audible signals based on those sensed conditions. The patient monitor (10) may simply provide an audible signal that is indicative of the patient heart beat and also has audible and/or visible alarms that are activated when a particular sensed parameter of condition falls outside certain settings. A remote device (30) is adapted to be located remote from the patient monitor (10), such as in an adjoining room (50), where the audible sound and/or light signals from the patient monitor (10) cannot be perceived sufficiently. The remote device (30) is in electrical communication (52) with the patient monitor (10) and produces a sound and/or light simultaneously with the sound and/or light of the patient monitor (10).

## Description

### Background

The present invention relates to a system and device for providing a remote signal or signals to a user, and, more particularly, to a system that allows a user, such as a caregiver, to receive audible and/or visual signals from a patient monitor at a location remote and possible isolated from the patient monitor itself.

In health care facilities, it is, of course, very common to have a patient monitor that basically provides a continual monitoring of certain conditions of the patient. In most cases, the monitor involves a visual display of signals and/or an audible sound. For example, the patient monitor may provide an alarm in the event some sensed parameter or condition of the patient is outside of certain set limits. Such event would cause the monitor to alert the attending caregiver by an audible and/or visible alarm that is built into the monitor itself.

Other functions of a patient monitor are in the providing of a continual or periodic report of a condition of the patient by, for example, an audible sound or a visable light. An example may be where the monitor is monitoring the heartbeat of the patient and there is a continual beeping from the patient monitor indicative of the heart beat of the patient and which provides the caregiver with a continuous recognition of the patient's heart rate. That heart beat sound may even be combined with the output from an oximeter, where the pitch of the sound may vary in accordance with the oxygenation of the patient's blood.

In other monitors, there are messages that are conveyed to the caregiver by a monitor display and those messages are prioritized in accordance with the urgency of the particular message. Thus, the messages are normally accompanied by an audible sound and/or visible light that signifies to the caregiver, the urgency of the message.

In any event, the visual and/or audible signals are provided by some built in devices into the patient monitor itself and therefore have a limited range of effectiveness, that is, they cannot be so loud as to annoy the patient or nearby patients or interfere with other patient monitors performing the same monitoring function.

As such, the effectiveness of the visual and/or audible signals from the patient monitor are therefore limited. The range of the visible signals require that the caregiver be within sight of the patient monitor and, with an audible signal, the caregiver must be within range of the volume of the sound emitted from the patient monitor.

Unfortunately, there are times when the caregiver is not physically in the room with the patient but may be outside that room with limited or no visual or audible access to the patient monitor. At those times, the monitoring function is compromised and there is the possibility of an alarm condition or other change in the patient condition that may not be effectively and rapidly communicated to the caregiver.

It would therefore be advantageous to have device and a system that provided a remote access by a caregiver to the alarms and other visual and audible functions of a patient monitor such that those audible and visual functions could be simultaneously provided at a remote location, general outside of the room where the patient is located, to provide the same information to a caregiver at a remote location that is provided by the patient monitor proximate to the patient.

### Summary of the Invention

Accordingly, with the present invention, there is provided a remote device and system that emits light and produces sounds that are indicative of the same function as are provided by the patient monitor located in close proximity to the patient. Those sound and light signals are produced by a remote device simultaneously with the sounds and light signals of the patient monitor and give the same information to a caregiver.

The remote device includes a light emitting device, such as one or more LEDs, and a sound producing device, such as a speaker, and those devices are in communication with the corresponding light or sound producing devices incorporated into the patient monitor. In one exemplary embodiment, the sound and light devices in the remote device are hard wired to the sound and light devices of the patient monitor.

As such, as the sound and/or light is produced at the patient monitor, the same sound and/or light is produced at the remote device and that remote device can then be located external of the room in which the patient is being treated, such that the caregiver can be attending to other duties or patients in an adjoining room and still be receiving the needed information regarding the condition of the patient sensed by the patient monitor.

As a further feature of the present invention, the present system can be retrofitted to existing patient monitors by simply attaching hard wires to the sound and light functions of the patient monitor and installing the remote device at a location outside the patient's room. Accordingly, the existing patient monitor can be modified to attach the hard wires internal of the monitor box or there may be installed a jack or other connection device that provides a connection to the wires leading to the remote device. As such, a jack receptacle can be built into the patient monitor such that the signals leading to the remote device can simply have a jack or jacks that plug into one or more receptacles on the box of the patient monitor. If both sound and light signals are transmitted to the remote device, there may be separate jacks for the differing types of signals.

As a further alternative embodiment, the light or sound signals from the patient monitor can be transmitted by some wireless protocol to the remote device located external of the room housing the patient.

As a further embodiment, there may be a plurality of remote devices that are situated in different rooms so that the caregiver has more range of location while still being able to recognize and comprehend the sound and light signals from the patient monitor.

These and other features and advantages of the present invention will become more readily apparent during the following detailed description taken in conjunction with the drawings herein.

### Brief Description of the Drawings

Figure 1 is a perspective view of a patient monitor currently available commercially;
Figure 2 is a perspective view of a typical remote device usable with the present invention; and
Fig. 3 is a schematic view of the system of the present invention illustrating the typical locations for the patient monitor and the remote device.

### Detailed Description of the Invention

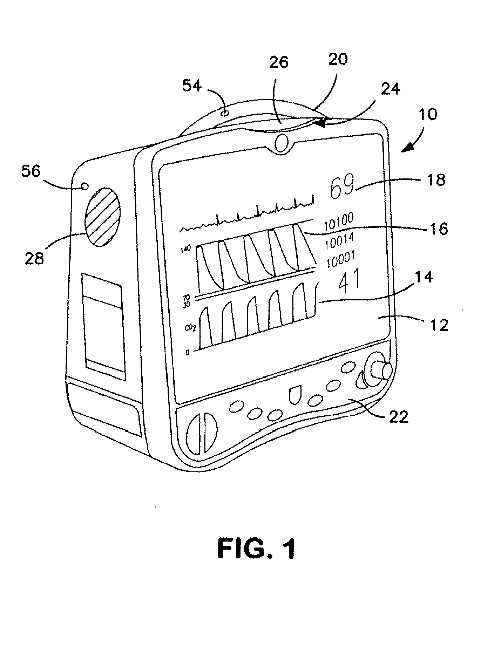

Referring now to Figure 1, there is shown a perspective view of a currently available commercial patient monitor 10. In describing the present invention, reference will be made to the Fig. 1 patient monitor illustrated as an exemplary embodiment and which is descriptive of the DASH ® Series of patient monitors available from the General Electric Company. As will be seen, however, the present system can be used with a wide variety of patient monitors consistent with the intent and purpose of the present invention.

In any event, taking the patient monitor 10 of Fig. 1, the patient monitor 10 has a display 12 that provides a reading to the caregiver of various monitored conditions and parameters. In Fig. 1, the display 12 includes a wave form 14 illustrating the CO₂ level in the patient airway, a wave form 16 indicative of non-invasive blood pressure and a wave form 18 illustrating ECG. The parameters are typical of those incorporated into a patient monitor, however, other sensed conditions may include pulse oximetry (SPO₂), temperature, respiration, invasive blood pressure as well as other conditions and parameters. As such, the present invention can be used with any number of differing configurations of patient monitors. The patient monitor 10 also includes a carrying handle 20 to facilitate moving the patient monitor 10 from one location to another and a control panel 22 that allows the user to selectively carry out the operation of the patient monitor 10.

In addition, in the patient monitor 10 of Fig. 1, there is a light emitting device 24 that includes a red LED and a yellow LED, not shown, that produce a light that passes out though a window 26 to provide information to the caregiver as to the condition or urgency of messages that are provided to the caregiver by the patient monitor 10. In addition, there is a sound producing device 28, shown for illustration purposes as a speaker, that produces sounds to be heard by the caregiver and which can also be indicative of the urgency of a message provided by the patient monitor 10 to that caregiver.

Both the light emitting device 24 and the sound producing device 28 are used to convey information to a caregiver. For example, the patient monitor 10 has messages that are conveyed to the caregiver and the messages are of differing levels of urgency. In general, there is a level one message where the patient monitor 10 simply displays a message to the user on the display 12. There is then a level two message that is of a higher urgency and that message is provided on the display 12 but brought to the attention of the caregiver by means of a single beeping sound produced by the sound producing device 28 and accompanied by a flashing yellow light visible at the light emitting device 24

There is a third level of urgency and that message is accompanied by two high pitched sounds produced by the sound producing device 28 along with a blinking yellow light visible at the light emitting device 24. Finally, for a message of the highest urgency or level four, the message is accompanied by a flashing red light visible at the light emitting device 24 as well as three high pitched sounds produced by the sound producing device 28.

As can therefore be seen, the patient monitor 10 makes use of both of light and sound to convey messages to the caregiver. In addition, the illustrated patient monitor 10 or another patient monitor, may also provide a sound in a form representative of the patient pulse or other continual sound that conveys some information to the caregiver relating to a condition of the patient and those sounds can be effectively used in carrying out the present invention.

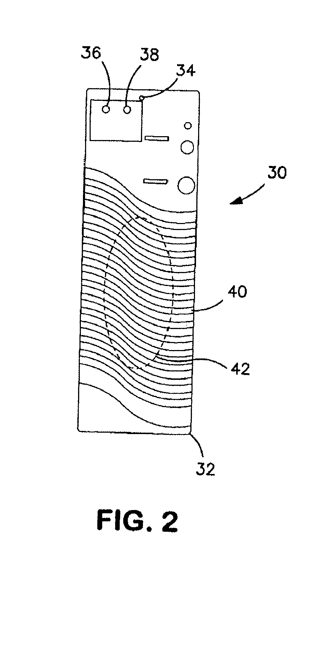

Turning now to Fig. 2, taken along with Fig. 1, there is shown a perspective view of a remote device 30 that is a component of the present invention. As can be seen, the remote device 30 comprises a housing 32 that is adapted to stand on a flat surface and which has a light emitting device 34 comprising a red LED 36 and a yellow LED 38. There is also an opening 40 in the remote device 30 so that sound can pass through the housing 32 from a sound producing device 42 such as a speaker located behind the opening 40.

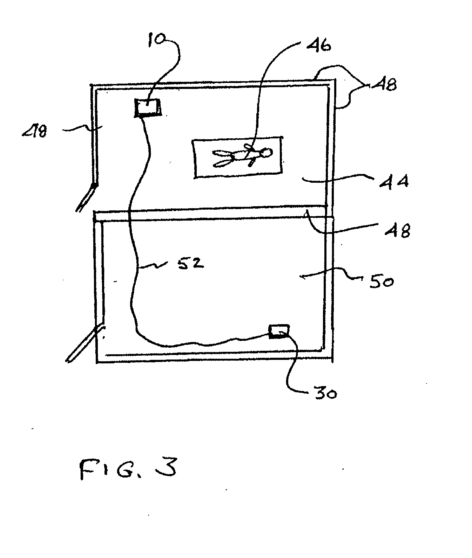

Turning finally to Fig. 3, taken along with Figs. 1 and 2, there is shown a schematic view of the system of the present invention with the patient monitor 10 in communication with the remote device 30. As can be seen, the patient monitor 10 is located in a patient room 44 along with the patient 46 whereas the patient monitor 10 is positioned proximate to the patient 46 and serves to monitor a function or condition of the patient 46.

The patient 46, along with the patient monitor 10 is located in the patient room 44 having walls 48 whereas the remote device 30 is located external of the patient room 44, for example, in an adjoining room 50. The adjoining room 50 may not have a view of the patient monitor 10 located within the patient room 44 such that a caregiver in the adjoining room 50 cannot visually perceive the patient monitor 10. Additionally, the walls 48 serve to isolate the caregiver from sounds and therefore prevent the caregiver from hearing any audible sounds produced by the patient monitor 10 such as have been previously described.

Accordingly, the presence of the remote device 30 in the adjoining room 50, that is hard wired to the patient monitor by means of wires 52, allows the sound and light from the patient monitor 10 to be remotely produced by the remote device 30, that is, the light is simultaneously emitted from the light emitting device 24 of the patient monitor 10 as well as the light emitting device 34 of the remote device 26 and the same is true of sound, that is, sound is produced by the sound producing device 42 of the remote device 30 simultaneously with the same sound being produced by the sound producing device 28 of the patient monitor 10. Thus, the caregiver, located in the adjoining room 50 has the benefit of seeing and hearing light and sound signals emitted by the remote device 30 that are also and simultaneously produced by the patient monitor 10.

The hard wiring together of the patient monitor 10 and the remote device 30 can be accomplished by simply connecting the wires 52, respectively, to both the light emitting devices 24 and 34 as well as the sound producing devices 28 and 42 and the wiring can readily be accomplished in a retrofit manner, that is, the present invention can be adapted for use with existing patient monitors by adding the hard wiring connection to the patient monitor and providing a remote device.

Alternatively, the hard wiring can be carried out by the addition of jack receptacles 54, 56 affixed to the patient monitor 10 (Fig. 1). The hard wiring of the light emitting devices can be facilitated by the jack receptacle 54 located on the carrying handle 20 and the sound producing hard wiring by the jack receptacle 56 located proximate to the sound producing device 28, however, any convenient location on the patient monitor 10 can be used for mounting the jack receptacles 54, 56. As such the hard wires 52 can simply have a respective jack affixed to their ends that are plugged into the jack receptacles 54, 56 to hard wire the remote device 30 to the patient monitor 10 and achieve the advantages of the present invention.

As a further alternative, the light and sound signals for the patient monitor 10 can be wirelessly transmitted to the remote device 30 by a wireless system using one of many protocols so that the need for hard wiring is eliminated.

Those skilled in the art will readily recognize numerous adaptations and modifications which can be made to the system of the present invention which will result in an improved remote monitoring system, yet all of which will fall within the scope and spirit of the present invention as defined in the following claims. Accordingly, the invention is to be limited only by the following claims and their equivalents.

Aspects of the present invention are defined in the following numbered clauses:
1. A system for providing remote access to audible and/or visual signals from a patient monitor, the system comprising a patient monitor adapted to be located proximate to a patient, the patient monitor having an audible device for producing an audible sound and/or a light device for emitting visible light, the light device and/or audible device being responsive to some parameter surrounding the patient or a sensed patient condition sensed by the patient monitor, and a remote device having the capability of providing visible and/or audible signals, the remote device being in electrical communication with the patient monitor wherein a visible and/or audible signal emitted from the patient monitor is simultaneously emitted by the remote device.
2. The system of clause 1, wherein the remote device is in electrical communication with the patient monitor by means of hard wires.
3. The system of clause 1 or clause 2, wherein the remote device has a light emitter and a sound emitter.
4. The system of clause 2, wherein the patient monitor has at least one jack receptacle that provides light and/or sound signals and the hard wires include at least one jack that is insertable into a jack receptacle to transmit sound and/or light signals to the remote device.
5. The system of clause 4, wherein the at least one jack receptacle comprises first and second jack receptacles, the first jack receptacle providing light signals and the second jack receptacle providing sound signals and the hard wires include first and second jacks, the first jack being insertable into the first jack receptacle and the second jack being insertable into the second jack receptacle so as to separately transmit both light and sound signal to the remote device.
6. The system of any one of the preceding clauses, wherein the patient monitor is an infant monitor adapted to sense and provide an audible sound indicative of heart rate.
7. The system of clause 6, wherein the remote device simultaneously with the infant monitor provides an audible sound indicative of heart rate.
8. The system of any one of the preceding clauses, wherein the patient monitor has at least one audible or visible alarm that is activated upon sensing an abnormal condition of a patient and the remote device simultaneous with the activation of that alarm, also provides an audible or visible alarm indicative of an abnormal condition of a patient.
9. The system of any one of the preceding clauses, wherein the patient monitor and the remote device simultaneously emit light and produce sound indicative of the priority of a message provided to a caregiver by a display on the patient monitor.
10. A method of creating a remote indication of a condition sensed by a patient monitor, the method comprising the steps of:
   providing a patient monitor and locating that patient monitor proximate to a patient within a room, the patient monitor having the ability to provide audible and/or visible signals to a caregiver indicative of a condition of a patient,
   providing a remote device having the ability to produce audible and/or visible signals at a location outside of the room where the patient is located,
   electrically communicating the remote device with the patient monitor, wherein the audible and/or visible signal is provided by the remote device simultaneously with the same audible and/or visible signal provided at the patient monitor.
11. The method of clause 10, wherein the step of electrically communicating the remote device with the patient monitor comprises hard wiring together the remote device and the patient monitor.
12. The method of clause 10 or clause 11, wherein the step of providing a patient monitor comprises providing a patient monitor having a sound producing device and the step of providing a remote device comprises providing a remote device having a sound producing device and the step of electrically communicating the remote device with the patient monitor comprises hard wiring the sound producing device of the patient monitor to the sound producing device of the remote device.
13. The method of any one of clauses 10 to 12, wherein the step of providing a patient monitor comprises providing a patient monitor having a light emitting device and the step of providing a remote device comprises providing a remote device having a light emitting device and the step of electrically communicating the remote device with the patient monitor comprises hard wiring the light emitting device of the patient monitor to the light emitting device of the remote device.
14. The method of clause 13, wherein the step of providing a patient monitor comprises providing a patient monitor having a sound producing device and a light emitting device and the step of providing a remote device comprises providing a remote device having a sound producing device and a light emitting device and the step of electrically communicating the remote device with the patient monitor comprises hard wiring the light emitting device of the patient monitor to the light emitting device of the remote device and the sound producing device of the patient monitor to the sound producing device of the remote device.
15. The method of any one of clauses 10 to 14, wherein the step of providing a patient monitor comprises providing a patient monitor having at least one jack receptacle for communication of sound and/ or light signals from the patient monitor.
16. The method of clause 15, wherein the step of providing a patient monitor comprises providing a patient monitor having at least two jack receptacles for separately communication of sound and or light signals from the patient monitor.
17. A remote device for receiving and providing audible and/or visible signals from a patient monitor, the remote device comprising a housing having a light emitting device and a sound producing device, the remote deice adapted to receive signals from a patient monitor located proximate to a patient and to emit light signals and produce sound signals simultaneously with light and sound signals produced by a patient monitor.
18. A remote device for receiving and providing audible and/or visible signals from a patient monitor as defined in clause 17, wherein the light emitting device comprises at least one of a red LED and a yellow LED.
19. A remote device for receiving and providing audible and/or visible signals from a patient monitor as defined in clause 18, wherein the light emitting device comprises a red LED and yellow LED.
20. A remote device for receiving and providing audible and/or visible signals from a patient monitor as defined in any one of clauses 17 to 19, wherein the sound producing device is a speaker enclosed with the housing.

## Claims

1. A system for providing remote access to audible and/or visual signals from a patient monitor (10), the system comprising a patient monitor (10) adapted to be located proximate to a patient (46), the patient monitor (10) having an audible device (28) for producing an audible sound and/or a light device (24) for emitting visible light, the light device (24) and/or audible device (28) being responsive to some parameter surrounding the patient (46) or a sensed patient condition sensed by the patient monitor (10), and a remote device (30) having the capability of providing visible and/or audible signals, the remote device (30) being in electrical communication with the patient monitor (10) wherein a visible and/or audible signal emitted from the patient monitor (10) is simultaneously emitted by the remote device (30).

2. The system of claim 1, wherein the remote device (30) is in electrical communication with the patient monitor (10) by means of hard wires (52).

3. The system of claim 1 or claim 2, wherein the remote device (30) has a light emitter (34) and a sound emitter (42).

4. The system of claim 2, wherein the patient monitor has at least one jack receptacle that provides light and/or sound signals and the hard wires include at least one jack that is insertable into a jack receptacle to transmit sound and/or light signals to the remote device.

5. The system of claim 2, wherein the patient monitor (10) includes first and second jack receptacles (54, 56), the first jack receptacle providing light signals (54) and the second jack receptacle (56) providing sound signals and further includes first and second jacks, the first jack being insertable into the first jack receptacle (54) and the second jack being insertable into the second jack receptacle (56) so as to separately transmit both light and sound signals to the remote device (30).

6. The system of any one of the preceding claims, wherein the patient monitor (10) is an infant monitor adapted to sense and provide an audible sound indicative of heart rate.

7. The system of claim 6, wherein the remote device (30) simultaneously with the infant monitor (10) provides an audible sound indicative of heart rate.

8. The system of any one of the preceding claims, wherein the patient monitor (10) has at least one audible or visible alarm (24, 28) that is activated upon sensing an abnormal condition of a patient (46) and the remote device (30) simultaneous with the activation of that alarm, also provides an audible or visible alarm (34, 42) indicative of an abnormal condition of a patient (46).

9. The system of any one of the preceding claims, wherein the patient monitor (10) and the remote device (30) simultaneously emit light and produce sound indicative of the priority of a message provided to a caregiver by a display (12) on the patient monitor (10).

10. A method of creating a remote indication of a condition sensed by a patient monitor (10), the method comprising the steps of:
providing a patient monitor (10) and locating that patient monitor(10) proximate to a patient (46) within a room (44), the patient monitor (10) having the ability to provide audible and/or visible signals (24, 28) to a caregiver indicative of a condition of a patient (46),
providing a remote device (30) having the ability to produce audible and/or visible signals (34, 42) at a location outside of the room (44) where the patient(46) is located,
electrically communicating the remote device (30) with the patient monitor (10), wherein the audible and/or visible signal is provided by the remote device (30) simultaneously with the same audible and/or visible signal provided at the patient monitor (10).

11. The method of claim 10, wherein the step of electrically communicating the remote device with the patient monitor comprises hard wiring together the remote device and the patient monitor.

12. The method of claim 10 or claim 11, wherein the step of providing a patient monitor comprises providing a patient monitor having a sound producing device and the step of providing a remote device comprises providing a remote device having a sound producing device and the step of electrically communicating the remote device with the patient monitor comprises hard wiring the sound producing device of the patient monitor to the sound producing device of the remote device.

13. The method of any one of claims 10 to 12, wherein the step of providing a patient monitor comprises providing a patient monitor having a light emitting device and the step of providing a remote device comprises providing a remote device having a light emitting device and the step of electrically communicating the remote device with the patient monitor comprises hard wiring the light emitting device of the patient monitor to the light emitting device of the remote device.

14. The method of claim 13, wherein the step of providing a patient monitor comprises providing a patient monitor having a sound producing device and a light emitting device and the step of providing a remote device comprises providing a remote device having a sound producing device and a light emitting device and the step of electrically communicating the remote device with the patient monitor comprises hard wiring the light emitting device of the patient monitor to the light emitting device of the remote device and the sound producing device of the patient monitor to the sound producing device of the remote device.

15. A remote device (30) for receiving and providing audible and/or visible signals from a patient monitor (10), the remote device (10) comprising a housing (32) having a light emitting device (34) and a sound producing device (42), the remote device (30) adapted to receive signals from a patient monitor (10) located proximate to a patient (46) and to emit light signals and produce sound signals simultaneously with light and sound signals produced by a patient monitor (10).
